(19) Europäisches Patentamt
European Patent Office
Office européen des brevets

(11) Veröffentlichungsnummer : **0 024 532**
**B2**

(12) # NEUE EUROPÄISCHE PATENTSCHRIFT

(45) Veröffentlichungstag der neuen Patentschrift : **15.08.84**

(21) Anmeldenummer : **80104184.9**

(22) Anmeldetag : **17.07.80**

(51) Int. Cl.$^3$ : **C 07 C 47/198**, C 07 C 43/10, C 07 C 45/50, C 07 C 41/18, A 61 K 7/46, C 11 D 3/50

(54) **Tert.-Butoxybutanale und -butanole sowie Bis-tert.-Butoxypentanale und -pentanole, deren Herstellung und Verwendung als Duftstoffe.**

(30) Priorität : **10.08.79 DE 2932527**

(43) Veröffentlichungstag der Anmeldung : **11.03.81 Patentblatt 81/10**

(45) Bekanntmachung des Hinweises auf die Patenterteilung : **23.06.82 Patentblatt 82/25**

(45) Bekanntmachung des Hinweises auf die Entscheidung über den Einspruch : **15.08.84 Patentblatt 84/33**

(84) Benannte Vertragsstaaten : **BE CH DE FR GB IT LI**

(56) Entgegenhaltungen :
**EP-A- 0 018 161**
**EP-A- 0 018 162**
**EP-A- 0 018 163**
**EP-A- 0 018 164**
**DE-A- 2 715 685**
**An article by E.L. Eliel et al., J. Org. Chem. Vol. 30, pages 2441 to 2447 (published July 1965)**
**An article by P.C. Loewen et al., Can. J. Chem., Vol. 50, pages 1502 to 1512 (1972)**

(73) Patentinhaber : **BASF Aktiengesellschaft**
**Carl-Bosch-Strasse 38**
**D-6700 Ludwigshafen (DE)**

(72) Erfinder : **Himmele, Walter, Dr.**
**Eichenweg 14**
**D-6909 Walldorf (DE)**
Erfinder : **Hoffmann, Werner, Dr.**
**Ringstrasse 11 C**
**D-6701 Neuhofen (DE)**

**EP 0 024 532 B2**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft tert.-Butoxybutanale und -butanole sowie Bis-tert.-Butoxypentanale und + pentanole der allgemeinen Formeln I und II

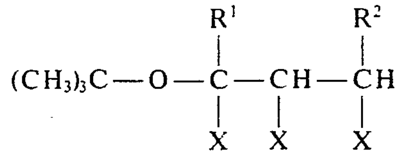

(I)

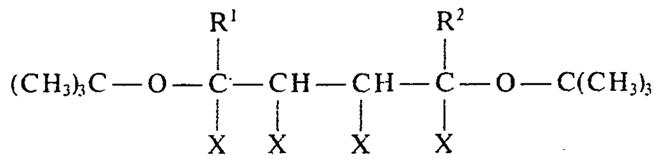

(II)

in denen die Reste $R^1$ und $R^2$ Wasserstoff oder $C_1$-$C_4$-Alkylgruppen und einer der Reste X eine Formylgruppe oder eine Hydroxymethylgruppe und die übrigen Reste X Wasserstoff bedeuten, ausgenommen 4-tert.-Butoxybutan-1-al, 4-tert.-Butoxybutan-1-ol, 2-Methyl-3-tert.-butoxypropan-1-al und 2-Methyl-3-tert.-butoxypropan-1-ol.

Die Ausnahmebestimmung am Ende der vorstehenden Definition gründet sich darauf, daß das 4-tert.-Butoxybutan-1-ol in J. Org. Chem., Band 30 (1965), Seite 2426, sowie in Can. J. Chem., Band 50 (1972), Seite 1510, bereits als Hydrierungsprodukt des 2-Butoxytetrahydrofurans erwähnt ist, und daß alle von der Ausnahmebestimmung umfaßten Verbindungen sowie auch deren Herstellung Gegenstand der offengelegten älteren Europäischen Patentanmeldungen EP-A1 0 018 161-64 sind. Die Bezugnahme auf die vom Schutzbegehren ausgenommenen Verbindungen sowie auf deren Herstellung wurde in der Offenbarung der vorliegenden Erfindung belassen, soweit dies zum Verständnis der Erfindung erforderlich ist.

Die definitionsgemäßen Aldehyde, also diejenigen Verbindungen I und II, in denen einer der Reste X eine Formylgruppe bedeutet, werden im folgenden als Verbindungen IA und IIA und die entsprechenden Alkohole, in denen X für eine Hydroxymethylgruppe steht, als Verbindungen IB und IIB bezeichnet.

Der Erfindung lag die Herstellung neuer Duftstoffe sowie neuer Zwischenprodukte für organische Synthesen zugrunde.

Es wurde gefunden, daß man die Aldehydether IA und IIA in bekannter Weise durch Hydroformylierung der entsprechenden Allylether (III) bzw. der Bisether der But-2-en-1,4-diole (IV)

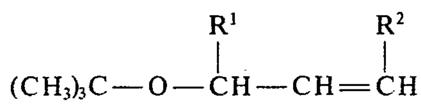

(III)

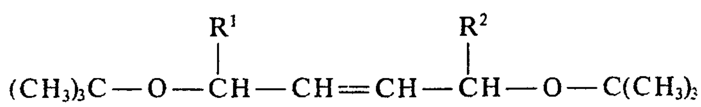

(IV)

in Gegenwart von Rhodiumkatalysatoren erhält.

Je nach den Reaktionsbedingungen und je nachdem, ob die Reste $R^1$ und $R^2$ Wasserstoff oder Alkylgruppen bedeuten, entstehen bei der Hydroformylierung von III und IV die gemäß den allgemeinen Formeln IA und IIA möglichen Isomeren IA/a, IA/b, IA/c, IIA/a, IIA/a', IIA/b und IIA/b'.

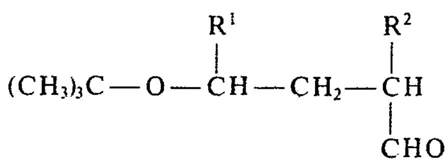

(IA/a) 4-tert.-Butoxy-butan-1-ale

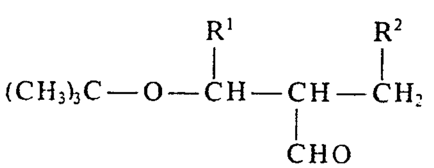

(IA/b) 3-tert.-Butoxy-2-methylpropan-1-ale
(Stammkörper Isobutanal)

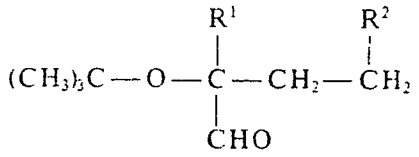

(IA/c) 2-tert.-Butoxy-butan-1-ale

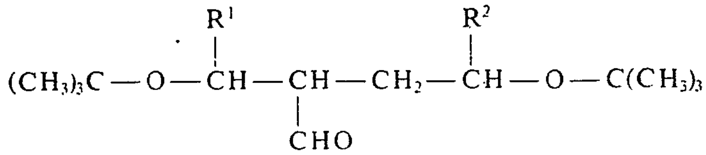

(IIA/a)

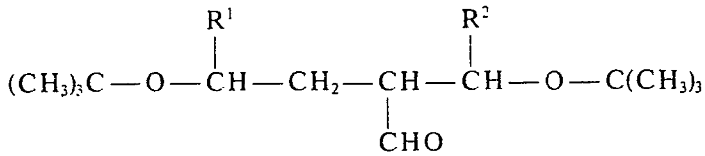

(IIA/a')

1,4-Bis-tert.-butoxybutan-2-ale (Stammkörper Isopentanale)

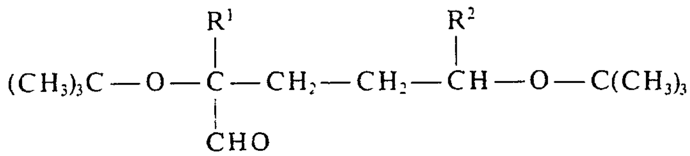

(IIA/b)

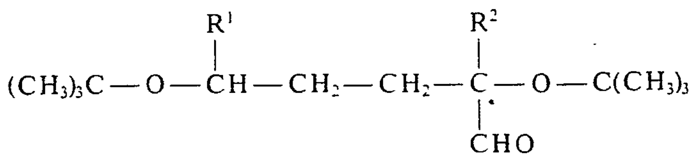

(IIA/b')

1,4-Bis-tert.-butoxypentan-2-ale

Sind die Reste $R^1$ und $R^2$ im Falle der Verbindungen IA Wasserstoff, worauf sich das Schutzbegehren nicht mehr erstreckt, so erhält man bei 50-700 bar und 50-100 °C, Reaktionszeiten von 2-12 Stunden sowie einer Katalysatorkonzentration von 50-1 000 ppm Rhodium, bezogen auf die Menge des Reaktionsgemisches sowie auf Rh-Metall, Isomerengemische aus Verbindungen vom Typ IA/a und IA/b im Verhältnis von etwa 70-30 % zu 45-55 %, während die Verbindung vom Typ IA/c nur in untergeordneter Menge anfällt. Unter den gleichen Reaktionsbedingungen, sowie unter der Voraussetzung, daß $R^1$ und $R^2$ für Wasserstoff als bevorzugte Reste stehen, erhält man aus IV fast ausschließlich die Verbindung vom Typ IIA/a (= IIA/a' wegen der Symmetrie) und daneben untergeordnete Mengen des isomeren Aldehyds vom Typ IIA/b.

Höhere oder überwiegende Anteile der Verbindungen vom Typ IA/c und IIA/b erhält man, sofern $R^1$ und $R^2$ Wasserstoff bedeuten, wenn man die Hydroformylierung der Ausgangsolefine bei 50-700 bar, 110-160 °C, einer Reaktionszeit von 0,5-6 Stunden und einer Katalysatorkonzentration von 10-200 ppm Rh vornimmt. Unter diesen Bedingungen findet eine verstärkte Olefin-Isomerisierung der Art

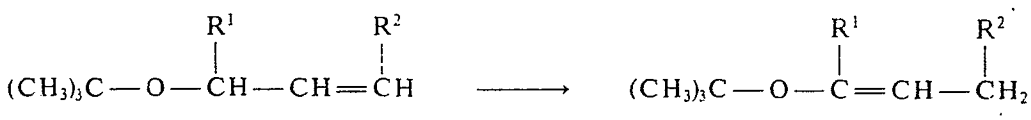

statt. Diese Isomerisierung kann man in einer vorgeschalteten Reaktion mittels eines Isomerisierungskatalysators bei 100-180 °C auch getrennt vornehmen, wobei sich als Isomerisierungskatalysator Pd und Cu sowie bevorzugt Rhodiumverbindungen in Konzentrationen von 50-1 000 ppm eignen. Die Hydroformylierung kann dann unter den Bedingungen ausgeführt werden, wie sie für die Herstellung der Verbindungen des Typs IA/a, IA/b und IIA/a genannt wurden.

Sind einer oder beide der Reste $R^1$ und $R^2$ Alkylgruppen, so gelten für die Herstellung der Verbindungen IA/a, IA/b, IIA/a und IIA/a' einerseits und der Verbindungen IA/c, IIA/b und IIA/b' andererseits die für die unsubstituierten Verbindungen angegebenen Reaktionsbedingungen, jedoch ändert sich hier das Isomerenverhältnis infolge des Einflusses der Substituenten nach der Regel von Keulemans, derzufolge die Formylgruppe bevorzugt an denjenigen C-Atomen eintritt, die noch die meisten H-Atome tragen und/oder am wenigsten sterisch behindert sind.

Die Ausgangsverbindungen III und IV sind bekannt oder in bekannter Weise durch Umsetzung der entsprechenden Alkohole V und VI

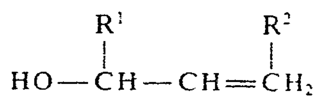

(V)

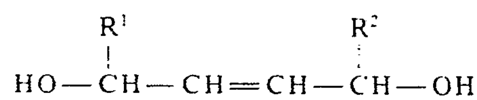

(VI)

mit Isobuten in Gegenwart saurer Katalysatoren erhältlich. Die wichtigsten Stammkörper V und VI, von denen sich die erfindungsgemäßen Verbindungen I und II ableiten lassen, sind

Allylalkohol ($R^1 = R^2 = H$), für die Herstellung von 2-tert.-Butoxybutanal und -butanol
But-1-en-3-ol ($R^1 = CH_3$ ; $R^2 = H$)
But-2-en-1-ol ($R^1 = H$ ; $R^2 = CH_3$)
But-2-en-1,4-diol ($R^1 = R^2 = H$)
Pent-2-en-1,4-diol ($R^1 = H$ ; $R^2 = CH_3$) und
Hex-3-en-2,4-diol ($R^1 = R^2 = CH_3$),

wobei sich im Falle isomerer Verbindungen jeweils die cis- und die trans-Form gleichermaßen eignen.

Sieht man von den oben angegebenen speziellen Hydroformylierungen für die Steuerung der Isomerenverhältnisse ab, nimmt man die Hydroformylierung wie üblich allgemein bei 50-700 bar, 50-160 °C, einer Reaktionsdauer von 3-6 Stunden und einer Katalysatorkonzentration von 10-1 000 ppm Rh vor. Vorzugsweise verwendet man hierbei ein äquimolares oder annähernd äquimolares Gemisch aus Kohlenmonoxid und Wasserstoff. Die Gegenwart eines Lösungsmittels ist nicht erforderlich, empfiehlt sich jedoch häufig, um z. B. ein homogenes Reaktionsmedium zu gewährleisten. Geeignete Lösungsmittel sind beispielsweise Diethylether, Dioxan und Tetrahydrofuran und Aromaten wie Toluol und Xylol.

Als Katalysatoren eignen sich Rh-Komplexe wie

$Rh_2(cyclooctadien)_2Cl_2$
$Rh_2(ethylen)_2Cl_2$
$Rh_2(CO)_2Cl_2$ und
$Rh_2(acetat)_4$.

Statt der Komplexverbindungen kann man auch sonstige Rh-Verbindungen wie Rhodiumoxid oder Rhodiumsalze wie Rhodiumacetat einsetzen, da sich die aktiven Carbonylkomplexe unter den Reaktionsbedingungen, gegebenenfalls zusammen mit weiteren getrennt zugegebenen Liganden wie Triphenylphosphin, in situ bilden.

Hydriert man die Verbindungen IA und IIA, so erhält man die entsprechenden Etheralkohole IB bzw. IIB, bei denen die Formylgruppe jeweils durch eine Hydroxymethylgruppe ersetzt ist. Man kann die Hydrierung nach allen hierfür bekannten Verfahrensweisen vornehmen, bevorzugt jedoch die katalytische Hydrierung mit Wasserstoff in Gegenwart von Raney-Nickel, Cr/Cu-oxid (Adkins-)-Katalysatoren oder von Edelmetallkatalysatoren wie Pt/Aktivkohle. Vorzugsweise führt man die Hydrierung in Methanol oder Ethanol als Lösungsmittel bei 20-200 °C und unter einem Druck von 1-200 bar aus.

Die Aufarbeitung auf die Verfahrensprodukte kann in üblicher Weise durch fraktionierte Destillation erfolgen, da sich die entstehenden Isomeren in ihren Siedepunkten unterscheiden.

Die erfindungsgemäßen tert.-Butoxybutanale und -butanole I sowie die Bis-tert.-butoxypentanale und -pentanole II sind wertvolle Duftstoffe von frisch-mentholhaltigem bis blumigem Geruch. Sie eigenen sich daher zur Herstellung von Kosmetika aller Art sowie auch zur Parfümierung von Waschmitteln, Reinigungsmitteln und ähnlichen im Haushalt und Gewerbe anwendbaren Präparaten und Chemikalien.

Ferner sind die Verbindungen I und II Zwischenprodukte für organische Synthesen.

Als unmittelbare Derivate von I und II sind hier vor allem die Aldehydalkohole sowie die Polyole VII und VIII hervorzuheben.

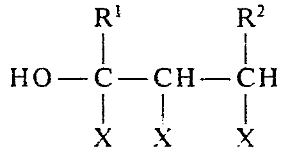

(VII)

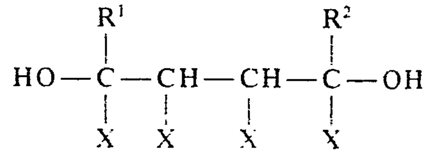

(VIII)

eines der X gleich $—CHO$ bzw. $—CH_2OH$, die übrigen H,
die man in bekannter Weise durch Abspaltung der tert.-Butoxygruppe mittels saurer Katalyse erhält.

## Beispiel 1

4-tert.-Butoxybutan-1-al, 2-Methyl-3-tert.-butoxypropan-1-al und 2-tert.-Butoxybutan-1-al

4 100 g Allyl-tert.-butylether wurden mittels 50 mg Dirhodium-dicyclooctadien-dichlorid bei 130 °C und 200 bar mittels eines äquimolaren $CO/H_2$-Gemisches im Laufe von 12 Stunden umgesetzt.

Anschließend wurden die flüchtigen Bestandteile des Reaktionsgemisches bei 50 mbar vom Katalysator sowie festen und hochsiedenden Rückständen abdestilliert, wobei 1 210 g Rückstand und 3 480 g Destillat anfielen. Da kein Allylether mehr nachweisbar war, errechnet sich in erster Näherung hieraus ein Umsatz von rund 74 %. Nach gaschromatischer Analyse entfielen davon, bezogen auf den Umsatz (= 100 %)

16 % auf das 4-tert.-Butoxybutan-1-al,
32 % auf das 2-Methyl-3-tert.-butoxypropan-1-al und
30 % auf das 2-tert.-Butoxybutan-1-al.

Der Rest bestand aus nicht weiter identifizierten Substanzen.

Die fraktionierte Destillation lieferte die drei isomeren tert.-Butoxybutanale in etwa den gleichen Ausbeuten wie nach der gaschromatographischen Analyse.

Die Kenndaten des 2-tert.-Butoxybutan-1-al sind : Sdp. 47 °C/14 mbar ; $n_D^{20}$ 1,397 7 ; Geruchscharakteristik : säuerlich, stechend ; nach 2 Stunden krautig.

## Beispiel 2

4-tert.-Butoxypentan-1-al und 2-Methyl-3-tert.-butoxybutan-1-al

3 500 g 3-tert.-Butoxybut-1-en wurden mittels 250 mg des in Beispiel 1 genannten Rh-Katalysators bei 60 °C und 650 bar mittels eines Äquimolaren $CO/H_2$-Gemisches im Laufe von 36 Stunden wie üblich hydroformyliert.

Die destillative Aufarbeitung des Reaktionsgemisches lieferte folgendes Ergebnis :

— 4-tert.-Butoxypentan-1-al ; Sdp. 73 °C/14 mbar ;
$n_D^{20}$ 1,424 2
Geruchscharakteristik : fettig, krautig
— 2-Methyl-3-tert.-butoxybutan-1-al ; Sdp. 67 °C/14 mbar ;
$n_D^{20}$ 1,418 6
Geruchscharakteristik : blumig, süßlich.

## Beispiel 3

2-Formyl-1,4-bis-tert.-butoxybutan

2 500 g 1,4-Bis-tert.-butoxybut-2-en wurden mittels 50 mg des in Beispiel 1 genannten Rh-Katalysators bei 100 °C und 650 bar mittels eines äquimolaren $CO/H_2$-Gemisches im Laufe von 24 Stunden in Gegenwart von 2 000 g Tetrahydrofuran wie üblich hydroformyliert.

Die fraktionierte Destillation des Reaktionsgemisches lieferte den oben genannten Aldehyd-bis-ether in einer absoluten Ausbeute von 79 %. Sdp. 77-78 °C/5 mbar.

## Beispiel 4

3-Formyl-2,5-bis-tert.-butoxyhexan

200 g 2,5-Bis-tert.-butoxyhex-3-en wurden mittels 100 mg des in Beispiel 1 genannten Rh-Katalysators bei 70 °C und 650 bar mittels eines äquimolaren $CO/H_2$-Gemisches im Laufe von 12 Stunden in Gegenwart von 200 g Toluol wie üblich hydroformyliert. Die fraktionierte Destillation des Reaktionsgemisches lieferte den oben genannten Aldehyd-bis-ether. Sdp. (geringe Zersetzung) 135-145 °C/4 mbar.

## Beispiel 5

4-tert.-Butoxynonan-1-al und 2-Methyl-3-tert.-butoxyoctan-1-al

Je 200 g 3-tert.-Butoxyoct-1-en wurden mittels 100 mg des in Beispiel 1 genannten Rh-Katalysators

a) bei 80 °C und
b) bei 110 °C

und 650 bar mittels eines äquimolaren $CO/H_2$-Gemisches im Laufe von 12 Stunden in Gegenwart von 200

g Tetrahydrofuran wie üblich hydroformyliert. Der gaschromatographisch ermittelte Umsatz der Ausgangsverbindung betrug bei 80 °C 84 % und bei 110 °C 91 %. Die übliche Aufarbeitung der Reaktionsgemische lieferte folgende Ergebnisse :

— 4-tert.-Butoxynonan-1-al
bei 80 °C Ausbeute (absolut) : 45 %
bei 110 °C Ausbeute (absolut) : 61 %
Sdp. : 74 °C/0,5 mbar ; $n_D^{20}$ 1,435 0
Geruchscharakteristik : blumig, jasminartig noch nach 24 Stunden

— 2-Methyl-3-tert.-butoxyoctan-1-al
bei 80 °C Ausbeute (absolut) : 28 %
bei 110 °C Ausbeute (absolut) : 29 %
Sdp. : 95-96 °C/13 mbar
Geruchscharakteristik : fettig.

Beispiel 6

2-tert.-Butoxybutan-1-ol

Eine Suspension aus 250 g Äthanol und 30 g eines Cu/Cr-oxid Hydrierkatalysator (Adkins-Kontakt) wurde zur Aktivierung des Kontaktes zunächst 12 Stunden lang unter einem Wasserstoffdruck von 100 bar auf 120 °C erhitzt.

Danach wurde die so vorbereitete Suspension bei 80 °C und 60 bar im Laufe von 6 Stunden mit 438 g 2-tert.-Butoxy-butanal versetzt und noch 12 Stunden unter diesen Bedingungen nachreagieren gelassen.

Die übliche destillative Aufarbeitung des vom Katalysator abgetrennten Reaktionsgemisches lieferte die oben genannte Verbindung.

Ausbeute : 83 %
Sdp. 108 °C/100 mbar ; $n_D^{20}$ 1,424 9
Geruchscharakteristik : mentholartig-minzig.

**Ansprüche**

1. Tert.-Butoxybutanale und -butanole sowie Bis-tert.-butoxypentanale und -pentanole der allgemeinen Formeln I und II

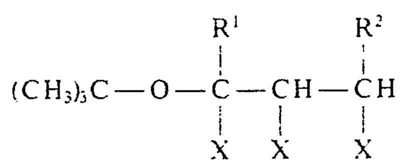
(I)

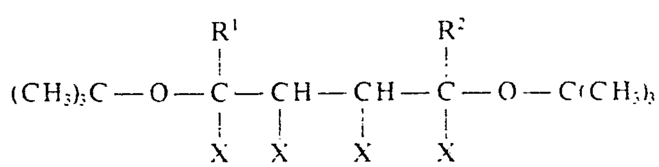
(II)

in denen die Reste $R^1$ und $R^2$ Wasserstoff oder $C_1$-$C_4$-Alkylgruppen und einer der Reste X eine Formylgruppe oder eine Hydroxymethylgruppe und die übrigen Reste X Wasserstoff bedeuten, ausgenommen 4-tert.-Butoxybutan-1-al, 4-tert.-Butoxybutan-1-ol, 2-Methyl-3-tert.-butoxy-propan-1-al und 2-Methyl-3-tert.-butoxy-propan-1-ol.

2. 2-Tert.-Butoxybutan-1-al.
3. 4-Tert.-Butoxypentan-1-al.
4. 2-Methyl-3-tert.-butoxybutan-1-al.
5. 2-Formyl-1,4-bis-tert.-butoxybutan.
6. 3-Formyl-2,5-bis-tert.-butoxyhexan.
7. 4-Tert.-Butoxynonan-1-al.
8. 2-Methyl-3-tert.-butoxyoctan-1-al.
9. 2-tert.-Butoxybutan-1-ol.
10. Verfahren zur Herstellung von tert.-Butoxybutanalen (IA) und Bis-tert.-butoxypentanalen (IIA), dadurch gekennzeichnet, daß man die Allylether (III) bzw. die Bisether der But-2-en-1,4-diole (IV)

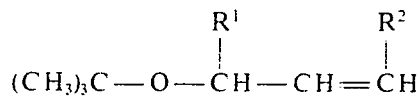

(III)

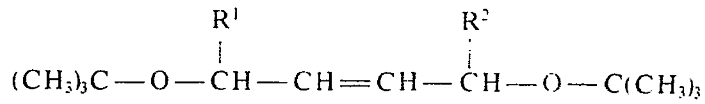

(IV)

in Gegenwart von Rhodiumkatalysatoren bei 50-160 °C und 50-700 bar mittels eines äquimolaren oder annähernd äquimolaren $CO/H_2$-Gemisches bei einer Katalysatorkonzentration von 10-1 000 ppm Rh in an sich bekannter Weise hydroformyliert, ausgenommen die Herstellung von 4-tert.-Butoxy butan-1-al und 2-Methyl-3-tert.-butoxy-propan-1-al aus tert.-Butyl-allyl ether.

11. Verfahren nach Anspruch 10, dadurch gekennzeichnet, daß man die Hydroformylierung zur Herstellung von größeren Anteilen der Isomeren IA/c, IIA/b und IIA/b'

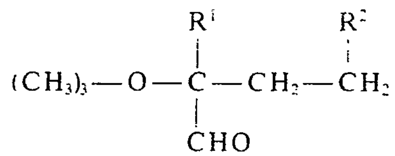

(IA/c)

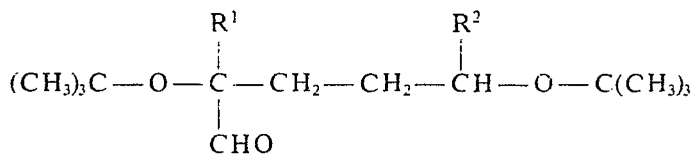

(IIA/b)

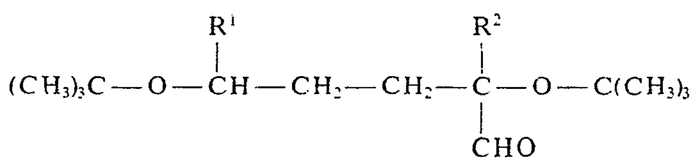

(IIA/b')

bei 110-160 °C und 50-700 bar in Gegenwart von 10-200 ppm des Rh-Katalysators, bezogen auf Rh-Metall und die Menge des Reaktionsgemisches, vornimmt.

12. Verfahren nach Anspruch 10, dadurch gekennzeichnet, daß man die Hydroformylierung zur Herstellung von größeren Anteilen der Isomeren IA/c, IIA/b und IIA/b' bei 50-100 °C und 50-700 bar in Gegenwart von 50-1 000 ppm des Rh-Katalysators, bezogen auf Rh-Metall und die Menge des Reaktionsgemisches, im Anschluß an eine Isomerisierungsreaktion in Gegenwart von 50-1 000 ppm Rh als Isomerisierungskatalysator bei 100-180 °C vornimmt.

13. Verfahren zur Herstellung von tert.-Butoxybutanolen (IB) und Bis-tert.-butoxypentanolen (IIB), dadurch gekennzeichnet, daß man die gemäß Anspruch 10 erhältlichen tert.-Butoxybutanale (IA) bzw. Bis-tert.-butoxypentanale (IIA) in an sich bekannter Weise hydriert, ausgenommen die Herstellung von 4-tert.-Butoxybutan-1-ol und 2-Methyl-3-tert.-butoxy-propan-1-ol.

**Claims**

1. Tert.-butoxybutanals and tert.-butoxybutanols, and bis-tert.-butoxypentanals and bis-tert.-butoxypentanols, respectively, of the general formulae I and II

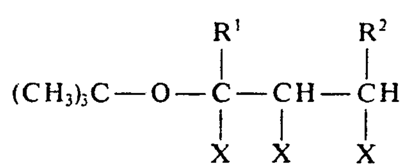

(I)

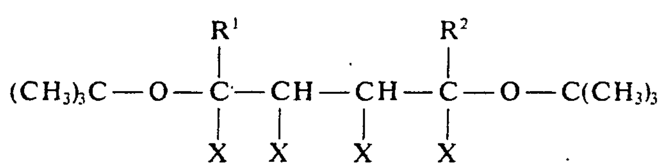

(II)

where $R^1$ and $R^2$ are hydrogen or $C_1$-$C_4$-alkyl, one of the radicals X is formyl or hydroxymethyl and the others are hydrogen, excluding 4-tert.-butoxybutan-1-al, 4-tert.-butoxybutan-1-ol, 2-methyl-3-tert.-butoxypropan-1-al and 2-methyl-3-tert.-butoxypropan-1-ol.

2. 2-tert.-Butoxybutan-1-al.

3. 4-tert.-Butoxypentan-1-al.

4. 2-Methyl-3-tert.-butoxybutan-1-al.

5. 2-Formyl-1,4-bis-tert.-butoxybutane.

6. 3-Formyl-2,5-bis-tert.-butoxyhexane.

7. 4-tert.-Butoxynonan-1-al.

8. 2-Methyl-3-tert.-butoxyoctan-1-al.

9. 2-tert.-Butoxybutan-1-ol.

10. A process for the preparation of tert.-butoxy-butanals (IA) and bis-tert.-butoxypentanals (IIA), characterized in that, respectively, an allyl ether (III) or a bis-ether of a but-2-ene-1,4-diol (IV)

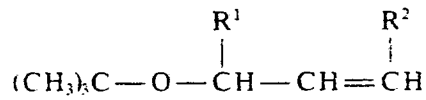

(III)

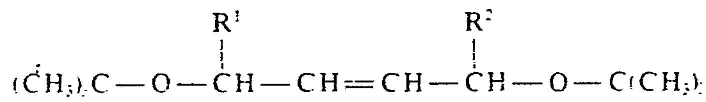

(IV)

is hydroformylated in a conventional manner by means of an equimolar or about equimolar mixture of CO and $H_2$ in the presence of a rhodium catalyst at 50-160 °C and 50-700 bar, using a catalyst concentration equivalent to 10-1 000 ppm of rhodium, excluding the preparation of 4-tert.-butoxybutan-1-al and 2-methyl-3-tert.-butoxypropan-1-al from tert.-butyl allyl ether.

11. A process as claimed in claim 10, characterized in that, in order to obtain higher proportions of the isomers IA/c, IIA/b and IIA/b'

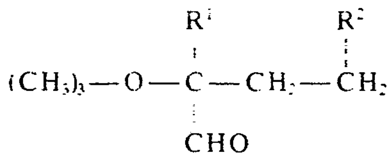

(IA/c)

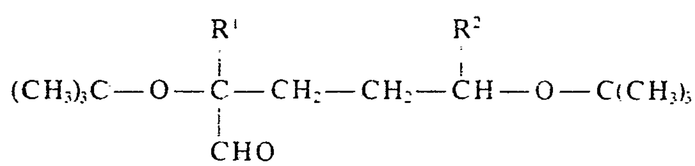

(IIA/b)

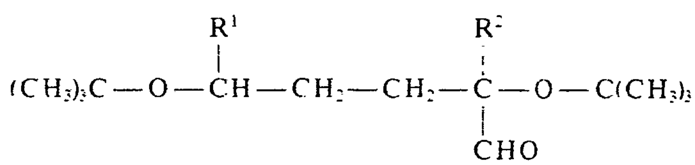

(IIA/b')

the hydroformylation is carried out at 110-160 °C and 50-700 bar in the presence of 10-200 ppm of the Rh catalyst, expressed as Rh metal and based in the amount of reaction mixture.

12. A process as claimed in claim 10, characterized in that, in order to obtain higher proportions of the isomers IA/c, IIA/b and IIA/b', the hydroformylation is carried out at 50-100 °C and 50-700 bar in the presence of 50-1 000 ppm of the Rh catalyst, expressed as Rh metal and based on the amount of reaction mixture, after having carried out an isomerization reaction in the presence of 50-1 000 ppm of rhodium as isomerization catalyst at 100-180 °C.

13. A process for the preparation of tert.-butoxybutanols (IB) and bis-tert.-butoxypentanols (IIB), characterized in that a tert.-butoxybutanal (IA) or bis-tert.-butoxypentanal (IIA) obtained by a process as claimed in claim 10 is hydrogenated in a conventional manner, excluding the preparation of 4-tert.-butoxybutan-1-ol and 2-methyl-3-tert.-butoxypropan-1-ol.

14. Use of the compounds I and II as claimed in claim 1 as fragrances for cosmetic preparations.

**Revendications**

1. Tert.-butoxy-butanals et -butanols, ainsi que bis-tert.-butoxy-pentanals et -pentanols des formules générales I et II

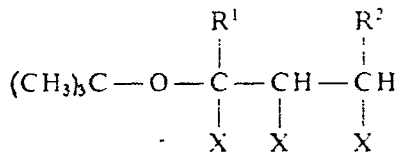

(I)

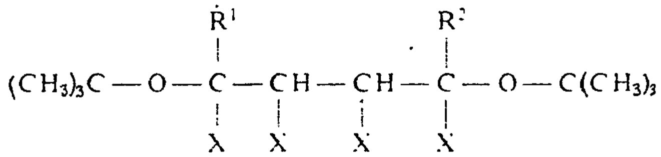

(II)

dans lesquelles les radicaux $R^1$ et $R^2$ représentent des atomes d'hydrogène ou des groupes alcoyle en $C_1$ à $C_4$ et l'un des radicaux X désigne un groupe formyle ou hydroxyméthyle, tandis que les autres radicaux X désignent des atomes d'hydrogène, à l'exception du 4-tert.-butoxy-butane-1-al, du 4-tert.-butoxy-butane-1-ol, du 2-méthyl-3-tert.-butoxy-propane-1-al et du 2-méthyl-3-tert.-butoxy-propane-1-ol.

2. Le 2-tert.-butoxy-butane-1-al.

3. Le 4-tert.-butoxy-pentane-1-al.

4. Le 2-méthyl-3-tert.-butoxy-butane-1-al.

5. Le 2-formyl-1,4-bis-tert.-butoxy-butane.

6. Le 3-formyl-2,5-bis-tert.-butoxy-hexane.

7. Le 4-tert.-butoxy-nonane-1-al.

8. Le 2-méthyl-3-tert.-butoxy-octane-1-al.

9. Le 2-tert.-butoxy-butane-1-ol.

10. Procédé de préparation de tert.-butoxy-butanals (IA) et de bis-tert.-butoxy-pentanals (IIA), caractérisé en ce que l'on soumet à une hydroformylation, de façon connue en soi, les éthers allyliques (III) ou les bis-éthers des but-2-ène-1,4-diols (IV)

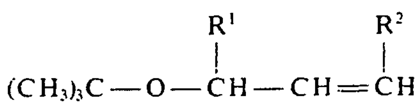

(III)

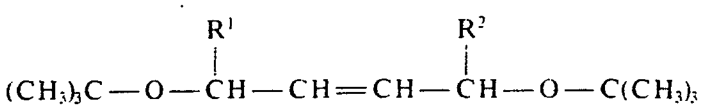

(IV)

entre 50 et 160 °C et sous 50 à 700 bars au moyen d'un mélange $CO/H_2$ équimolaire ou approximativement équimolaire, en présence de catalyseurs au rhodium, la concentration du catalyseur étant de 10 à 1 000 ppm de Rh, étant cependant exceptée la préparation du 4-tert.-butoxybutane-1-al et du 2-méthyl-3-tert.-butoxy-propane-1-al à partir d'éther butyl-allylique.

11. Procédé selon la revendication 10, caractérisé en ce que, pour l'obtention de proportions plus élevées en isomères IA/c, IIA/b et IIA/b'

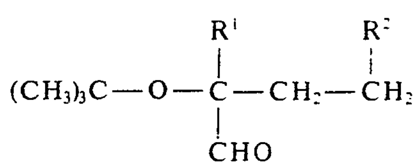

(IA/c)

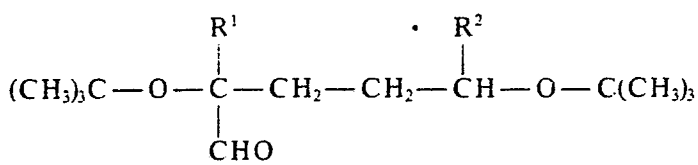

(IIA/b)

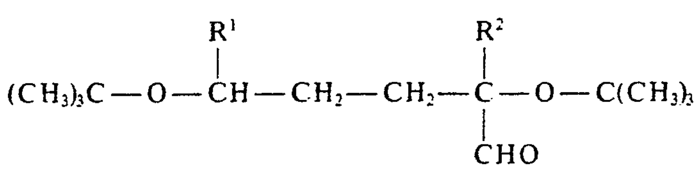

(IIA/b')

on procède à l'hydroformylation entre 110 et 160 °C et sous 50 à 700 bars en présence de 10 à 200 ppm du catalyseur au rhodium, calculé comme Rh métallique, par rapport au mélange réactionnel.

12. Procédé selon la revendication 10, caractérisé en ce que, pour l'obtention de proportions plus élevées en isomères IA/c, IIA/b et IIA/b', on procède à l'hydroformylation entre 50 et 100 °C et sous 50 à 700 bars en présence de 50 à 1 000 ppm du catalyseur au rhodium, calculé comme Rh métallique, par rapport au mélange réactionnel, cette opération faisant suite à une réaction d'isomérisation, réalisée entre 100 et 180 °C en présence de 50 à 1 000 ppm de Rh agissant comme catalyseur d'isomérisation.

13. Procédé de préparation de tert.-butoxy-butanols (IB) et de bis-tert.-butoxy-pentanols (IIB), caractérisé en ce que l'on soumet les tert.-butoxy-butanals (IA) ou bis-tert.-butoxy-pentanals (IIA) obtenus par le procédé selon la revendication 10, de façon connue en soi à une hydrogénation, étant cependant exceptée la préparation du 4-tert.-butoxy-butane-1-ol et du 2-méthyl-3-tert.-butoxy-propane-1-ol.

14. Utilisation des composés I et II selon la revendication 1 comme substances odoriférantes pour des compositions cosmétiques.